# EUROPÄISCHE PATENTSCHRIFT

(11) **EP 2 674 096 B1**
(45) Veröffentlichungstag und Bekanntmachung des Hinweises auf die Patenterteilung: **12.02.2020**
(21) Anmeldenummer: 13002818.6
(22) Anmeldetag: 31.05.2013
(51) Int. Cl.: A61B 1/00, G02B 23/24

(54) **Optisches Instrument**
Optical instrument
Instrument optique

(30) Priorität: 14.06.2012 DE 102012011717
(43) Veröffentlichungstag der Anmeldung: 18.12.2013
(73) Patentinhaber: Karl Storz SE & Co. KG, 78532 Tuttlingen (DE)
(72) Erfinder: Dahmen, Jan, 78606 Seitingen-Oberflacht (DE)
(74) Vertreter: Hofmeister, Frank

(56) Entgegenhaltungen:
- JP-A- H07 199 090
- US-A- 3 071 129
- US-A- 5 459 609
- US-A1- 2005 027 168
- US-A1- 2008 281 158

## Beschreibung

Die Erfindung betrifft ein optisches Instrument, insbesondere Endoskop, mit einem hohlen Instrumentengehäuse zur Aufnahme optischer Elemente, wobei das Instrumentengehäuse distalseitig und/oder proximalseitig über ein Endfenster fluiddicht verschlossen ist, wobei jedes der Endfenster jeweils über mindestens ein federbelastetes Dichtungselement gegenüber dem Instrumentengehäuse fluiddicht abgedichtet ist und wobei das mindestens eine federbelastete Dichtungselement jeweils über mindestens ein Federelement in Richtung auf jedes der Endfenster federbelastet ist, wobei in Axialrichtung des Instrumentengehäuses vor und hinter jedem der Endfenster jeweils ein federbelastetes Dichtungselement angeordnet ist, und die beiderseitigen Dichtungselemente über jeweilige Federelemente in Richtung auf das Endfenster federbelastet sind.

Endoskope und andere medizinische optische Instrumente müssen vor jedem Einsatz sterilisiert werden. Diese Sterilisation erfolgt heutzutage mittels Autoklavierung, wobei die optischen Instrumente einer Behandlung mit Heißdampf bei über 3 bar Druck und einer Temperatur von über 130° C ausgesetzt werden.

Um sicherzustellen, dass hohen thermischen Belastung keine Feuchtigkeit in das Instrumentengehäuse eindringt, ist es aus der Praxis bekannt, die als Deckgläser und/oder Endlinsen verwendeten endseitigen Endfenster durch Kleben, Schweißen oder Löten fluiddicht mit dem Instrumentengehäuse zu verbinden.

Ein gattungsgemäßes optische Instrument ist beispielsweise aus der US 5 459 609 A bekannt. Diese bekannte optische Baugruppe weist ein Basisteil, einen Deckel sowie ein zwischen diesen beiden Bauteilen angeordnetes Fenster auf, wobei zwischen den Bauteilen Basisteil und Deckel sowie dem Fenster beidseitig des Fensters Dichtungselemente angeordnet sind, die im montierten Zustand durch beiderseitige Federelemente federbelastet gegen die beiderseitigen Oberflächen des Fensters gedrückt werden.

Das federnde Verpressen der beiden Bauteile Basisteil und Deckel erfolgt über einen seitlichen elastischen Flansch des Deckels, der beim Verspannen von Basisteil und Deckel in Richtung des massiv ausgebildeten Flansches des Basisteils gebogen wird und so eine Federkraft auf die Dichtungselemente und das Fenster ausübt.

Nachteilig an dieser bekannten Konstruktion ist, dass der als Federelement dienende biegsame Flansch kein separates Bauteil ist, sondern Bestandteil des Deckels, weshalb eine individuelle Anpassung der erforderlichen Federkraft ohne eine konstruktive Änderung des Deckels kaum zu bewerkstelligen ist. Darüber hinaus ist das auf Seiten des Basisteils angeordnete Dichtungselement nur mittelbar federbelastet ausgebildet.

Weiterhin ist aus der US 2005/0027168 A1 ein Otoskop bekannt, bei dem wirkt die Federkraft einer Wendelfeder über eine Linse und einen Abstandshalter auf eine das Endfenster bildende Linse einwirkt und diese Linse proximalseitig abdichtend gegen eine O-Ring-Dichtung drückt.

Neben dem Ausgleich thermischer Spannungen beim Autoklavieren des optischen Instruments dienen die Federelemente insbesondere dazu einen über die Lebensdauer der Dichtungselemente gleichbleibenden Anpressdruck der Dichtungselemente gegen die jeweiligen Endfenster zu erzeugen. Insbesondere proximalseitig angeordnete Dichtungselemente sind beim Autoklavieren einer hohen Belastung ausgesetzt, weshalb es hier zu Undichtigkeiten kommen kann.

Darüber hinaus ist aus der US 2008/0281158 A1 ein Instrumentengehäuse bekannt, das über ein als Endlinse ausgebildetes Endfenster verschlossen ist. Ein Dichtungselement ist zwischen einem Flansch des Fensterrahmens und dem Flansch des Befestigungselement angeordnet und über ein Federelement federbelastet. Die federbelastete Dichtung wirkt somit nicht auf das Endfenster, sondern auf den Fensterrahmen. Die Endlinse ist bei dieser bekannten Konstruktion im Fensterrahmen durch Kleben, Schweißen oder Löten festgelegt. Hierbei kommt es aber aufgrund unterschiedlicher thermischer Ausdehnungskoeffizienten der verwendeten Materialien immer wieder zu erheblichen Spannungen, die zu Rissbildungen und/oder Spannungsbrüchen und somit zu Undichtigkeiten führen können

Ein weiteres optisches Instrument ist aus der DE 195 07 205 C2 bekannt. Aufgrund unterschiedlicher thermischer Ausdehnungskoeffizienten der verwendeten Materialien kommt es jedoch immer wieder zu erheblichen Spannungen, die zu Rissbildungen und/oder Spannungsbrüchen und somit zu Undichtigkeiten führen können.

Weiterhin ist aus der DE 699 23 388 T2 ein autoklavierbares Endoskop bekannt. Um das Instrumentengehäuse während des Autoklavierens gegen das Eindringen von Feuchtigkeit zu schützen, sind eine wasserdichte Kappe oder eine Rückschlagventilkappe vorgesehen, die am Optikgehäuse festlegbar sind.

Zwar gewährleisten diese zusätzlichen Abdeckkappen eine zuverlässige Abdichtung des Instrumentengehäuses, jedoch weist diese bekannte Konstruktion die Nachteile auf, dass einerseits ein separates Bauteil bereitgehalten werden muss und andererseits das Abdichten des Instrumentengehäuses eines zusätzlichen Montageschrittes bedarf, nämlich dem des Aufsetzens der Abdeckkappe.

Davon ausgehend liegt der Erfindung die **Aufgabe** zugrunde, ein optisches Instrument zu schaffen, das bei einfacher Montage eine zuverlässige fluiddichte Abdichtung des Instrumentengehäuses auch beim Autoklavieren gewährleistet.

Die **Lösung** dieser Aufgabenstellung ist erfindungsgemäß durch das Kennzeichen von Anspruch 1 definiert.

Durch die Verwendung eines federbelasteten Dichtungselements, also der Kombination eines klassischen Dichtungselements, wie beispielsweise einem O-Ring, mit einem auf das Dichtungselement einwirkenden Federelement wird ein definierter Anpressdruck des jeweiligen Endfensters gegen das Instrumentengehäuse erzielt, der die erforderliche Fluiddichtigkeit gewährleistet.

Die Elastizität des Federelements sowie des Dichtungselements gleichen die beim Autoklavieren auftretenden thermischen Spannungen ohne das Auftreten von Undichtigkeiten aus.

Neben dem Ausgleich der thermischen Spannungen dienen die Federelemente dazu einen über die Lebensdauer der Dichtungselemente gleichbleibenden Anpressdruck der Dichtungselemente gegen die jeweiligen Endfenster zu erzeugen. Da sich die Materialeigenschaften von Kunststoffen an Dichtungselementen über die Lebensdauer der Dichtungselemente verändern können und mehr oder weniger dazu neigen zu fließen, könnte sich der Anpressdruck der Dichtungselemente gegen die Endfenster im laufe der Zeit verringern, wenn nicht durch die erfindungsgemäßen Federelemente ein definierter Anpressdruck auf die Dichtungselemente ausgeübt würde.

Um die Dichtkraft an jedem fluiddicht abzudichtenden Endfenster zu verstärken, ist in Axialrichtung des Instrumentengehäuses vor und hinter jedem Endfenster mindestens jeweils ein Dichtungselement angeordnet, wobei die beiderseitigen Dichtungselemente jeweils über mindestens ein Federelement in Richtung auf das jeweilige Endfenster federbelastet sind.

Mit einer ersten Ausführungsform der Erfindung wird vorgeschlagen, dass die Federelemente unmittelbar am zugehörigen Dichtungselement anliegen.

Alternativ wird mit einer zweiten nicht zur Erfindung gehörigen Ausführungsform vorgeschlagen, dass die Federelemente jeweils über mindestens ein Druckelement auf das zugehörige Dichtungselement einwirken, wobei das mindestens eine Druckelement beispielsweise als Druckhülse ausgebildet ist.

Zur Ausbildung der Federelemente wird mit der Erfindung vorgeschlagen, dass die Federelemente als Federscheiben oder Federhülsen ausgebildet sind, wobei die Federscheiben vorzugsweise einseitig in einer radial umlaufenden Nut, beispielsweise im Instrumentengehäuse, gelagert sind.

Schließlich wird mit der Erfindung vorgeschlagen, dass die Endfenster als Deckglas und/oder Endlinse ausgebildet sind.

Weitere Merkmale und Vorteile der Erfindung ergeben sich anhand der zugehörigen Zeichnungen, in denen Ausführungsbeispiele eines optischen Instruments nur beispielhaft dargestellt sind, ohne die Erfindung auf diese Ausführungsbeispiele zu beschränken. In den Zeichnungen zeigt:
- Fig. 1: einen schematischen Längsschnitt durch ein als Endoskop ausgebildetes optisches Instrument gemäß dem Stand der Technik;
- Fig. 2: einen schematischen Schnitt durch das proximale Ende eines erfindungsgemäßen optischen Instruments;
- Fig. 3: eine Abbildung gemäß Fig. 2, eine zweite Ausführungsform darstellend, die jedoch nicht Teil der Erfindung ist;
- Fig. 4: eine Abbildung gemäß Fig. 2, eine dritte Ausführungsform darstellend, die jedoch nicht Teil der Erfindung ist;
- Fig. 5: einen schematischen Schnitt durch das distale Ende eines erfindungsgemäßen optischen Instruments, eine vierte Ausführungsform darstellend, die jedoch nicht Teil der Erfindung ist;
- Fig. 6: eine Abbildung gemäß Fig. 2, eine fünfte Ausführungsform darstellend, die jedoch nicht Teil der Erfindung ist und
- Fig. 7: eine vergrößerte Ansicht des Details VII gemäß Fig. 6.

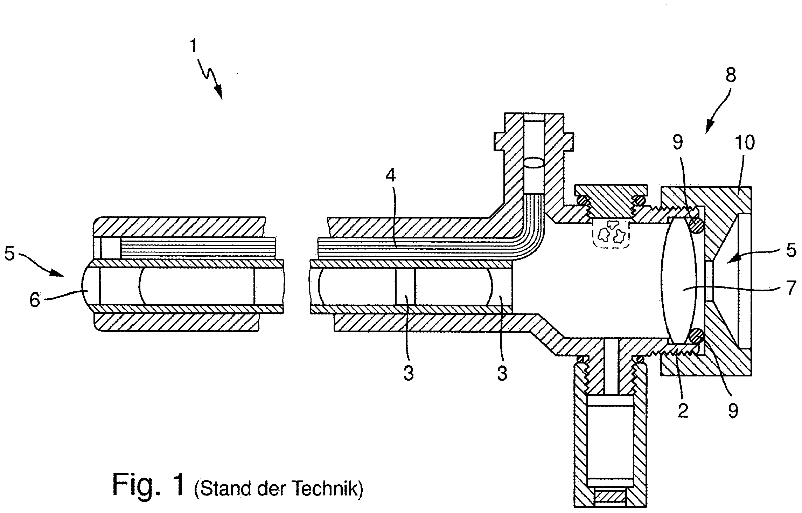

Die Abbildung Fig. 1 zeigt schematisch den Aufbau eines als Endoskop ausgebildeten optischen Instruments 1 gemäß dem Stand der Technik. Das optische Instrument 1 weist ein hohles Instrumentengehäuse 2 auf, in dem verschiedene optische Elemente, wie beispielsweise mehrere Linsen 3 und ein Bündel Lichtleiterfasern 4 angeordnet sind.

Distalseitig und proximalseitig ist das Instrumentengehäuse 2 über jeweils ein Endfenster 5 fluiddicht verschlossen. Bei dem in Fig. 1 dargestellten optischen Instrument 1 ist das distalseitige Endfenster 5 als Endlinse 6 und das proximalseitige Endfenster 5 als Deckglas 7 einer Okulareinheit 8 ausgebildet. Die endseitige Fluiddichtigkeit des Instrumentengehäuses 2 wird bei den aus dem Stand der Technik bekannten optischen Instrumenten 1 dadurch erzielt, dass die Endfenster 5 durch Verkleben, Verlöten oder durch den Einsatz von Dichtungselementen 9 im Instrumentengehäuse 2 festgelegt werden.

Beim Autoklavieren der optischen Instrumente 1 treten aufgrund unterschiedlicher thermischer Ausdehnungskoeffizienten der verwendeten Materialien insbesondere bei starr mit dem Instrumentengehäuse 2 verklebten oder verlöteten Endfenstern 5 erhebliche Spannungen auf, die zu Rissbildungen und/oder Spannungsbrüchen und somit zu Undichtigkeiten führen können. Aber auch elastische Dichtungselemente 9, wie beispielsweise O-Ringe 9, altern unter den beim Autoklavieren verwendeten Bedingungen derart, dass die Elastizität der Dichtungselemente 9 im Laufe der Zeit abnimmt, was wiederum zu Undichtigkeiten führen kann.

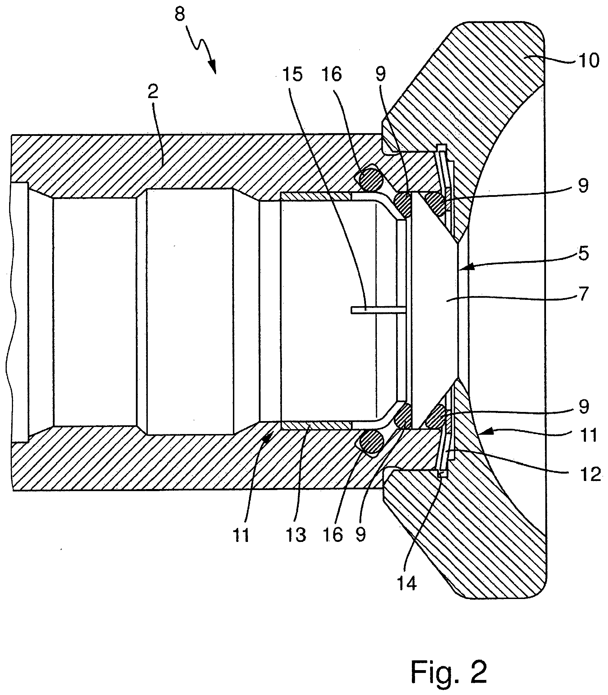

Bei den in den Abbildungen Fig. 2 bis Fig. 7 dargestellten optischen Instrumenten 1 erfolgt die fluiddichte Abdichtung zwischen den proximalseitigen und/oder distalseitigen Endfenstern 5 und dem Instrumentengehäuse 2 über federbelastete Dichtungselemente 9, die vorzugsweise als O-Ringe 9 ausgebildet sind.

Die Abbildung 2 zeigt schematisch den Aufbau einer Okulareinheit 8 eines optischen Instruments 1, wie beispielsweise eines Endoskops für medizinische oder nichtmedizinische Zwecke.

Die dargestellte Okulareinheit 8 besteht im Wesentlichen aus einer auf dem proximale Ende des Instrumentengehäuses 2 angeordneten Okularmuschel 10 und dem das Instrumentengehäuse 2 proximalseitig fluiddicht verschließenden, als Deckglas 7 ausgebildeten Endfenster 5.

Die Fluiddichtigkeit zwischen dem Deckglas 7 und dem Instrumentengehäuse 2 bewirken bei der dargestellten Okulareinheit 8 als O-Ringe 9 ausgebildete federbelastete Dichtungselemente 9. Bei der in Fig. 2 dargestellten ersten Ausführungsform sind in Axialrichtung des Instrumentengehäuses 2 vor und hinter dem Deckglas 7 als O-Ringe 9 ausgebildete Dichtungselemente 9 angeordnet, die jeweils über ein Federelement 11 in Richtung auf das Deckglas 7 federbelastet sind.

Durch die Federelemente 11 werden die Dichtungselemente 9 immer mit einem ausreichenden Dichtungsdruck gegen das Deckglas 7 gedrückt, so dass auch beim Auftreten thermischer Längenänderungen immer eine fluiddichte Abdichtung gewährleistet ist. Darüber hinaus gleichen die Federelemente 11 durch ihre Vorspannung einen möglichen Elastizitätsverlust der Dichtungselemente 9 aus, der aufgrund der Materialbeanspruchung im Laufe der Zeit bei den Dichtungselementen 9 auftreten kann.

Gemäß der in Fig. 2 dargestellten Ausführungsform sind die die Dichtungselemente 9 in Dichtrichtung vorspannenden Federelemente 11 als proximalseitig in der Okularmuschel 10 gelagerte Federscheibe 12 und distalseitig koaxial auf dem Instrumentengehäuse 2 gelagerte Federhülse 13 ausgebildet.

Zur Aufnahme und Lagerung der Federscheibe 12 ist in der Okularmuschel 10 eine radial umlaufende Nut 14 ausgebildet, in der die Federscheibe 12 einseitig gelagert ist. Mit dem radial innenliegenden Rand liegt die Federscheibe 12 so an dem als O-Ring 9 ausgebildeten Dichtungselement 9 an, dass dieses in Richtung auf das Deckglas 7 vorgespannt ist.

Die das distalseitige Dichtungselement 9 mit Federkraft beaufschlagende Federhülse 13 ist an ihrem proximalen Ende radial nach innen gekröpft und mit in Axialrichtung verlaufenden Schlitzen 15 versehen ausgebildet, um so eine radial nach außen auf das zughörige Dichtungselement 9 wirkende Federkraft ausüben zu können.

Wie weiterhin aus Fig. 2 ersichtlich, ist im Instrumentengehäuse 2 als Trockenmittel 16 eine hygroskopische Substanz angeordnet, um im Gehäuseinneren anfallende Feuchtigkeit zu binden, bevor sich diese auf den Linsen 3 niederschlägt. Auch, wenn das optische Instrument 1 durch die federbelasteten Dichtungselemente 9 zur Umgebung fluiddicht ausgebildet ist, kann sich beispielsweise bereits bei der Fertigung des optischen Instruments 1 in normaler Atmosphäre die Restfeuchte der Atmosphärenluft im Gehäuseinneren niederschlagen, die später zu einer Trübung der Linsen 3 führen könnte.

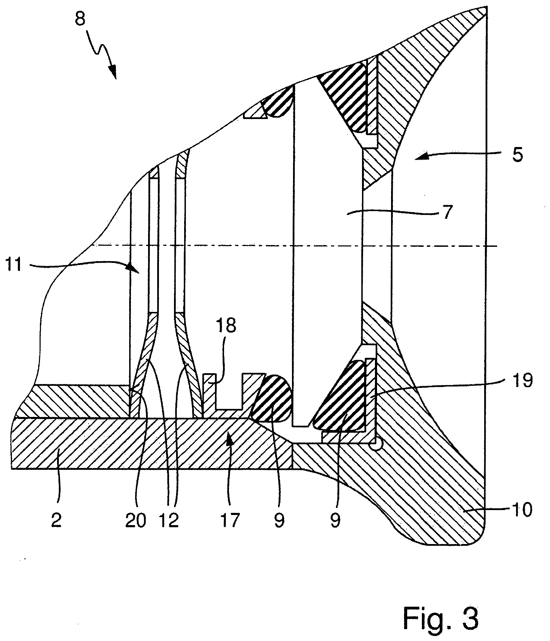

Die Anordnung des Trocknungsmittels 16 ist in Fig. 2 nur rein exemplarisch dargestellt. Das Trocknungsmittel 16 kann auch an einer anderen Stelle im Instrumentengehäuses 2 angeordnet sein und auch eine beliebige andere Form aufweisen. Weiterhin sei darauf hingewiesen, dass auch bei den nachfolgend in den Abbildungen Fig. 3 bis Fig. 7 dargestellten Ausführungsformen jeweils Trocknungsmittel 16 im Inneren der Instrumentengehäuse 2 angeordnet sein können, die Darstellung aber aus Gründen einer besseren Übersicht nur in Fig. 2 skizziert wurde.

Fig. 3 zeigt eine zweite Ausführungsform (die jedoch nicht Teil der Erfindung ist) zur Ausbildung einer fluiddichten Abdichtung zwischen einem Endfenster 5 und dem Instrumentengehäuse 2 ebenfalls am Beispiel einer Okulareinheit 8.

Wie aus Fig. 3 ersichtlich, sind auch bei dieser Ausführungsform in Axialrichtung des Instrumentengehäuses 2 betrachtet vor und hinter dem Deckglas 7 als O-Ringe 9 ausgebildete Dichtungselemente 9 angeordnet.

Im Gegensatz zur Ausführungsform gemäß Fig. 2, bei der die die Dichtungselemente 9 in Dichtungsrichtung vorspannenden Federelemente 11 direkt an den jeweiligen Dichtungselementen 9 anliegen, erfolgt bei der zweiten Ausführungsform die Übertragung der Federkraft auf die Dichtungselemente 9 nicht unmittelbar, sondern über mindestens ein zwischengelagertes Druckelement 17, beispielsweise eine Druckhülse 18.

Der Aufbau der fluiddichten Abdichtung dieser Ausführungsform sieht vor, dass das proximalseitige Dichtungselement 9 in einer radial nach innen zeigenden Abwinklung 19 des Instrumentengehäuses 2 angeordnet ist und vom sich in Axialrichtung nach distal anschließenden Deckglas 7 in dieser Lage gehalten wird. Distalseitig schließt sich an das Deckglas 7 das zweite Dichtungselement 9 an.

Zur Erzeugung der erforderlichen Dichtkraft ist in Axialrichtung weiter nach distal ein aus zwei Federscheiben 12 gebildetes Federelement 11 angeordnet. Die beiden Federscheiben 12 sind dabei so ausgebildet, dass ihre radial außen liegenden Ränder in Axialrichtung voneinander beabstandet sind und federelastisch aufeinander zu zusammendrückbar sind. Dadurch, dass sich die eine Federscheibe 12 an einem Widerlager 20 des Instrumentengehäuses 2 abstützt und die andere Federscheibe 12 an einer axial verschiebbaren Druckhülse 18 anliegt, wird über die Druckhülse 18 die nach proximal gerichtete Federkraft des Federelements 11 auf die beiden Dichtungselemente 9 ausgeübt. Über die Druckhülse 18 wird die Federkraft unmittelbar auf das distalseitige Dichtungselement 9 übertragen, welches über die Druckhülse 18 abdichtend gegen das Deckglas 7 gedrückt wird. Durch diese in proximale Richtung auf das Deckglas 7 ausgeübte Druckkraft wird das proximalseitige Dichtungselement 9 über das Deckglas 7 abdichtend in die Abwinklung 19 des Instrumentengehäuses 2 gepresst.

Aufgrund dieses skizzierten Aufbaus werden über das eine Federelement 11 beide Dichtungselemente 9 so mit einer Federkraft beaufschlagt, dass das Deckglas 7 das Instrumentengehäuse 2 proximalseitig fluiddicht verschließt.

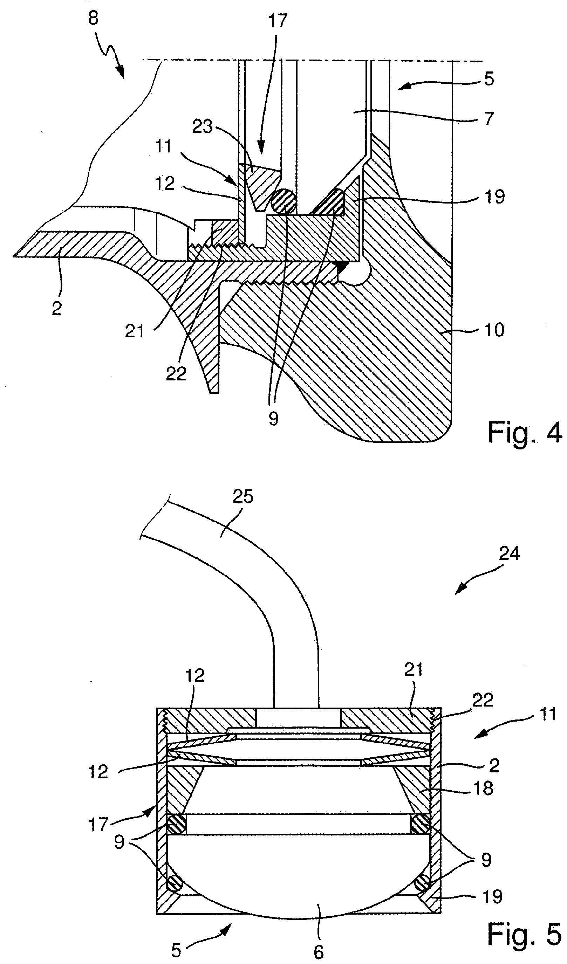

Die in Fig. 4 dargestellte dritte Ausführungsform (die jedoch nicht Teil der Erfindung ist) zur Ausbildung einer fluiddichten Abdichtung zwischen einem Endfenster 5 und dem Instrumentengehäuse 2 zeigt wiederum eine Okulareinheit 8.

Von der zuvor anhand der Abbildung Fig. 3 dargestellten Ausführungsform unterscheidet sich diese Ausgestaltung dadurch, dass das Federelement 11 aus nur einer Federscheibe 12 besteht, die mit ihrem radial außen liegenden Rand auf einem Schraubring 21 gelagert ist, der seinerseits über eine Gewindestrecke 22 in Axialrichtung des Instrumentengehäuses 2 verfahrbar ist.

Mit ihrem vom Schraubring 21 radial nach innen ragenden Teil liegt die Federscheibe 12 an einem Druckstück 23 an, das seinerseits am distalseitigen Dichtungselement 9 anliegt.

Durch Verfahren des Schraubrings 21 in die proximale Richtung wird die sich am Schraubring 21 abstützende Federscheibe 12 mit einer nach proximal gerichteten Federkraft beaufschlagt, die über das Druckstück 23 auf die beiden Dichtungselemente 9 wirkt. Über das Druckstück 23 wird die Federkraft unmittelbar auf das distalseitige Dichtungselement 9 übertragen, welches über das Druckstück 23 abdichtend gegen das Deckglas 7 gedrückt wird. Durch diese in proximale Richtung auf das Deckglas 7 ausgeübte Druckkraft wird das proximalseitige Dichtungselement 9 über das Deckglas 7 abdichtend in die Abwinklung 19 des Instrumentengehäuses 2 gepresst.

Aufgrund dieses skizzierten Aufbaus werden über die Federscheibe 12 beide Dichtungselemente 9 so mit einer Federkraft beaufschlagt, dass das Deckglas 7 das Instrumentengehäuse 2 proximalseitig fluiddicht verschließt. Über den Schraubring 21 ist bei dieser Ausgestaltungsform die über die Federscheibe 12 auf die Dichtungselemente 9 einwirkende Abdichtkraft einstellbar und wenn erforderlich nachstellbar.

Die in Fig. 5 dargestellte vierte Ausführungsform (die jedoch nicht Teil der Erfindung ist) zur Ausbildung einer fluiddichten Abdichtung zwischen einem Endfenster 5 und dem Instrumentengehäuse 2 zeigt die Abdichtung zwischen einer distalseitigen Endlinse 6 und dem Instrumentengehäuse 2 am Beispiel eines als Beleuchtungseinheit 24 ausgebildeten optischen Instruments 1.

Proximalseitig weist diese Beleuchtungseinheit 24 kein Endfenster 5, sondern einen Anschluss für ein Lichtleiterkabel 25 auf, über das Licht, ausgehend von einer nicht dargestellten Lichtquelle, zur Beleuchtungseinheit 24 übertragen wird.

Die Fluiddichtigkeit zwischen der Endlinse 6 und dem Instrumentengehäuse 2 bewirken bei der dargestellten Beleuchtungseinheit 24 als O-Ringe 9 ausgebildete federbelastete Dichtungselemente 9. Bei der in Fig. 5 dargestellten Ausführungsform sind in Axialrichtung des Instrumentengehäuses 2 vor und hinter der Endlinse 6 als O-Ringe 9 ausgebildete Dichtungselemente 9 angeordnet.

Der Aufbau der fluiddichten Abdichtung dieser Ausführungsform sieht vor, dass das distalseitige Dichtungselement 9 in einer radial nach innen zeigenden Abwinklung 19 des Instrumentengehäuses 2 angeordnet ist und von der sich in Axialrichtung nach proximal anschließenden Endlinse 6 in dieser Lage gehalten wird. Proximalseitig schließt sich an die Endlinse 6 das zweite Dichtungselement 9 an.

Die Abwinklung 19, die die Endlinse 6 in distaler Richtung teilweise überragt, dient neben der Ausbildung einer Anlagefläche für das distalseitige Dichtungselement 9 auch als Kratzschutz für die Endlinse 6.

Die Erzeugung der zur fluiddichten Abdichtung erforderlichen Dichtkraft erfolgt bei der Ausführungsform gemäß Fig. 5 über ein proximalseitig der Endlinse 6 angeordnetes, aus zwei Federscheiben 12 bestehendes Federpaket, dessen Federkraft nicht unmittelbar auf die Dichtungselemente 9 wirkt, sondern über eine zwischengelagerte Druckhülse 18.

Die beiden Federscheiben 12 sind dabei so ausgebildet, dass ihre radial außen liegenden Ränder aufeinander zu gebogen ausgebildet sind, so dass die Federscheiben 12 nur mit ihren radial außen liegenden Ränder aneinander anliegen und im Mittelbereich federelastisch voneinander beabstandet sind.

Dadurch, dass die proximalseitige der beiden Federscheiben 12 als Widerlager auf einem Schraubring 21 gelagert ist, der seinerseits über eine Gewindestrecke 22 in Axialrichtung des Instrumentengehäuses 2 verfahrbar ist, und die andere Federscheibe 12 an einer axial verschiebbaren Druckhülse 18 anliegt, wird über die Druckhülse 18 die nach proximal gerichtete Federkraft des Federpakets auf die beiden Dichtungselemente 9 ausgeübt. Über die Druckhülse 18 wird die Federkraft unmittelbar auf das proximalseitige Dichtungselement 9 übertragen, welches über die Druckhülse 18 abdichtend gegen die Endlinse 6 gedrückt wird. Durch diese in distale Richtung auf die Endlinse 6 ausgeübte Druckkraft wird das distalseitige Dichtungselement 9 über die Endlinse 6 abdichtend in die Abwinklung 19 des Instrumentengehäuses 2 gepresst.

Durch Verfahren des Schraubrings 21 in die distale Richtung werden die beiden Federscheiben 12 zusammengedrückt, wodurch eine nach distal gerichtete Federkrafterzeugt wird, die über die Druckhülse 18 auf die beiden Dichtungselemente 9 wirkt.

Aufgrund dieses skizzierten Aufbaus werden über die beiden Federscheiben 12 des Federpakets beide Dichtungselemente 9 so mit einer Federkraft beaufschlagt, dass die Endlinse 6 das Instrumentengehäuse 2 proximalseitig fluiddicht verschließt. Über den Schraubring 21 ist bei dieser Ausgestaltungsform die über die Federscheiben 12 auf die Dichtungselemente 9 einwirkende Abdichtkraft einstellbar und wenn erforderlich nachstellbar.

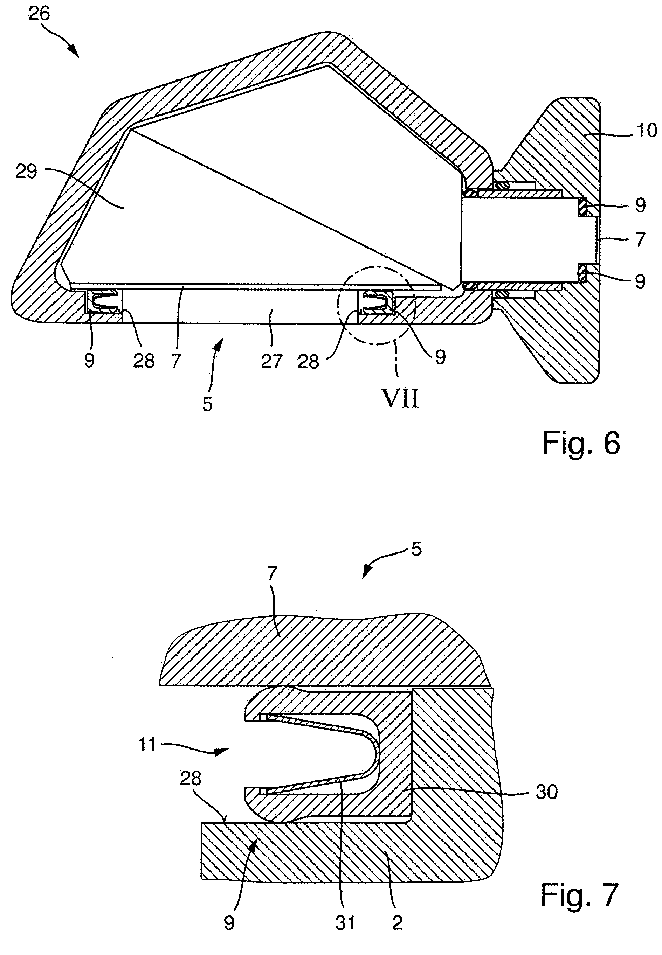

Die in Fig. 6 und 7 dargestellte fünfte Ausführungsform (die jedoch nicht Teil der Erfindung ist) zur Ausbildung einer fluiddichten Abdichtung zwischen einem Endfenster 5 und dem Instrumentengehäuse 2 zeigt die Abdichtung zwischen einem distalseitigen Deckglas 7 und dem Instrumentengehäuse 2 am Beispiel eines als Übersichtsoptik 26 ausgebildeten optischen Instruments 1.

Proximalseitig weist die Übersichtsoptik 26 eine Okularmuschel 10 auf, deren als Deckglas 7 ausgebildetes Endfenster 5 über nicht näher dargestellte, vorzugsweise als O-Ringe 9 ausgebildete Dichtungselemente 9 gegenüber dem Instrumentengehäuse 2 beispielsweise so abgedichtet ist, wie dies voranstehend anhand der in den Abbildungen Fig. 2, 3 und 4 dargestellten Ausführungsbeispiele beschrieben wurde.

Die distalseitige Fluiddichtigkeit zwischen dem Deckglas 7 und dem Instrumentengehäuse 2 bewirken bei der dargestellten Übersichtsoptik 26 federbelastete Dichtungselemente 9, deren genauerer Aufbau der vergrößerten Darstellung gemäß Fig. 7 zu entnehmen ist. Bei der in Fig. 6 und 7 dargestellten Ausführungsform ist nur distalseitig vor dem Deckglas 7 ein federbelastetes Dichtungselement 9 angeordnet.

Der Aufbau der fluiddichten Abdichtung dieser Ausführungsform sieht vor, dass das distalseitige federbelastete Dichtungselement 9 auf einem eine distalseitige Öffnung 27 des Instrumentengehäuses 2 umschließenden umlaufenden, als Rücksprung ausgebildeten Absatz 28 im Instrumentengehäuse 2 angeordnet ist und von dem sich nach proximal anschließenden Deckglas 7 in dieser Lage auf dem Absatz 28 gehalten wird. Proximalseitig schließt sich an das Deckglas 7 ein im Instrumentengehäuse 2 festgelegtes Prisma 29 an, wobei das Deckglas 7 und das Prisma 29 unmittelbar aneinander anliegen. Bedarfsweise kann die Kontaktfläche zwischen dem Deckglas 7 und dem Prisma 29 auch mit einem optischen Gel benetzt sein.

Zur Erzeugung der für die fluiddichte Abdichtung erforderlichen Dichtkraft ist bei dieser Ausführungsform, wie insbesondere aus Fig. 7 ersichtlich, das auf dem Absatz 28 angeordnete federbelastete Dichtungselement 9 als im Querschnitt U-förmige Dichtung 30 ausgebildet, wobei zwischen den parallelen Schenkeln der U-förmigen Dichtung 30 ein, beispielsweise aus Federstahl hergestelltes, als V-förmige Feder 31 ausgebildetes Federelement 11 angeordnet ist, dessen Federkraft unmittelbar auf das Dichtungselement 9 wirkt.

Die Verwendung dieser U-förmigen Dichtung 30 mit der V-förmige Feder 31 zur Ausbildung des federbelasteten Dichtungselements 9 hat den Vorteil, dass die Dichtung 30 mit der darin festgelegten V-förmigen Feder 31 als eine Baueinheit vorgefertigt werden kann, bevor diese Baueinheit in das Instrumentengehäuse 2 eingesetzt wird. Die U-förmige Dichtung 30 besteht vorzugsweise aus einem gummiartigen Material, jedoch sind auch weiche Kunststoffmaterialien zur Ausbildung dieser Dichtung 30 verwendbar.

Die V-förmige Feder 31 ist dabei so ausgebildet und in der U-förmigen Dichtung 30 angeordnet, dass dessen beiden am Dichtungselement 9 anliegenden Schenkel eine radial nach außen und schräg nach unten gerichtete Kraftkomponente auf das Dichtungselement 9 ausüben. Durch diese unmittelbar auf das Dichtungselement 9 ausgeübte Druckkraft wird das Dichtungselement 9 abdichtend gegen das Deckglas 7 gepresst. Da das Deckglas 7 proximalseitig als Widerlager unverrückbar am Prisma 29 anliegt, ist es bei dieser Ausführungsform ausreichend, nur distalseitig des Deckglases 7 ein federbelastetes Dichtungselement 9 anzuordnen.

Alternativ zu der in Fig. 6 und 7 dargestellten Ausführungsform, bei der das Instrumentengehäuse 2 distalseitig über ein Deckglas 7 verschlossen wird, ist es jedoch auch möglich dieses Deckglas 7 weg zu lassen, so dass das Instrumentengehäuse 2 distalseitig direkt durch das Prisma 29 verschlossen wird. Bei dieser Ausführungsform liegt das auf dem Absatz 28 des Instrumentengehäuses 2 angeordnete, als U-förmige Dichtung 30 mit innenliegender V-förmiger Feder 31 ausgebildete federbelastete Dichtungselement 9 das Instrumentengehäuse 2 fluiddicht abdichtend unmittelbar am Prisma 29 an.

Obwohl in den Abbildungen Fig. 2 bis Fig. 7 als Federelemente 11 überwiegend Federscheiben 12 und Federhülsen 13 dargestellt und beschrieben wurden, sind selbstverständlich auch andere Ausbildungen der Federelemente 11 möglich und einsetzbar, wenn sich mit diesen die zuvor beschriebene Wirkung, nämlich die Erzeugung eines ausreichenden Anpressdrucks auf die Dichtungselemente 9, bewerkstelligen lässt.

Alle voranstehend beschriebenen optischen Instrumente 1 zeichnen sich dadurch aus, dass die proximalseitigen und/oder distalseitigen Endfenster 5 jeweils über mindestens ein federbelastetes Dichtungselement 9 gegenüber dem Instrumentengehäuse 2 fluiddicht abgedichtet sind.

Die Elastizität des Federelements 11 sowie des Dichtungselements 9 gleichen die beim Autoklavieren auftretenden thermischen Spannungen ohne das Auftreten von Undichtigkeiten aus.

Neben dem Ausgleich der thermischen Spannungen dienen die Federelemente 11 dazu, einen über die Lebensdauer der Dichtungselemente 9 gleichbleibenden Anpressdruck der Dichtungselemente gegen die jeweiligen Endfenster 5 zu erzeugen.

## Patentansprüche

1. Optisches Instrument, insbesondere Endoskop, mit einem hohlen Instrumentengehäuse (2) zur Aufnahme optischer Elemente (3, 4), wobei das Instrumentengehäuse (2) distalseitig oder proximalseitig über ein Endfenster (5) fluiddicht verschlossen ist, wobei das Endfenster (5) über mindestens ein federbelastetes Dichtungselement (9) gegenüber dem Instrumentengehäuse (2) fluiddicht abgedichtet ist, wobei das mindestens eine federbelastete Dichtungselement (9) über mindestens ein Federelement (11) in Richtung auf das Endfenster (5) federbelastet ist, wobei in Axialrichtung des Instrumentengehäuses (2) vor und hinter dem Endfenster (5) jeweils ein federbelastetes Dichtungselement (9) angeordnet ist, wobei die beiderseitigen Dichtungselemente (9) über jeweilige Federelemente (11) in Richtung auf das Endfenster (5) federbelastet sind,
**dadurch gekennzeichnet, dass**
die Federelemente als außenliegende Federscheibe (12) und als innenliegende, koaxial auf dem Instrumentengehäuse (2) gelagerte Federhülse (13) ausgebildet sind.

2. Optisches Instrument nach Anspruch 1, **dadurch gekennzeichnet, dass** jedes der Federelemente (11) unmittelbar am zugehörigen Dichtungselement (9) anliegt.

3. Optisches Instrument nach einem der Ansprüche 1 bis 2, **dadurch gekennzeichnet, dass** die Dichtungselemente (9) als O-Ringe (9) ausgebildet sind.

4. Optisches Instrument nach Anspruch 1, **dadurch gekennzeichnet, dass** das Endfenster am proximalseitigen Ende des Instrumentengehäuses liegt, welches eine Okularmuschel (10) aufweist, in der eine radial umlaufende Nut (14) ausgebildet ist, in der die Federscheibe (12) einseitig gelagert ist.

5. Optisches Instrument nach einem der Ansprüche 1 bis 4, **dadurch gekennzeichnet, dass** das Endfenster (5) als Deckglas (7) und/oder Endlinse (6) ausgebildet ist.

## Claims

1. Optical instrument, in particular an endoscope, with a hollow instrument housing (2) for receiving optical elements (3, 4), wherein the instrument housing (2) is closed in a fluid-tight manner at the distal end or proximal end via an end window (5), wherein the end window (5) is sealed off in a fluid-tight manner with respect to the instrument housing (2) via at least one spring-loaded sealing element (9), wherein the at least one spring-loaded sealing element (9) is spring-loaded in the direction of the end window (5) via at least one spring element (11), wherein a respective spring-loaded sealing element (9) is arranged in front of and behind the end window (5) in the axial direction of the instrument housing (2), wherein the sealing elements (9) at both sides are spring-loaded in the direction of the end window (5) via respective spring elements (11), **characterized in that** the spring elements (11) are designed as an outer spring disc (12), and as an inner spring sleeve (13) mounted coaxially on the instrument housing (2).

2. Optical instrument according to Claim 1, **characterized in that** each of the spring elements (11) bears directly on the associated sealing element (9).

3. Optical instrument according to either of Claims 1 and 2, **characterized in that** the sealing elements (9) are designed as O-rings (9).

4. Optical instrument according to Claim 1, **characterized in that** the end window (5) lies at the proximal end of the instrument housing (2) having an eyepiece cup (10), in which a radially surrounding groove (14) is formed in which the spring disc (12) is mounted at one end.

5. Optical instrument according to one of Claims 1 to 4, **characterized in that** the end window (5) is designed as a cover glass (7) and/or end lens (6).

## Revendications

1. Instrument optique, en particulier un endoscope, avec un boîtier d'instrument (2) creux destiné à recevoir des éléments optiques (3, 4), dans lequel le boîtier d'instrument (2) est fermé de façon étanche aux fluides du côté distal ou du côté proximal par une fenêtre d'extrémité (5), dans lequel la fenêtre d'extrémité (5) est rendue étanche aux fluides à l'égard du boîtier d'instrument (2) par au moins un élément d'étanchéité (9) sollicité par ressort, dans lequel l'au moins un élément d'étanchéité (9) sollicité par ressort est sollicité par ressort dans la direction de la fenêtre d'extrémité (5) par au moins un élément de ressort (11), dans lequel un élément d'étanchéité (9) sollicité par ressort respectif est agencé devant et derrière la fenêtre d'extrémité (5) dans la direction axiale du boîtier d'instrument (2), dans lequel les éléments d'étanchéité (9) des deux côtés sont sollicités par ressort dans la direction de la fenêtre d'extrémité (5) par des éléments de ressort (11) respectifs,
**caractérisé en ce que**
les éléments de ressort (11) sont formés en tant que rondelle de ressort (12) extérieure et en tant que douille de ressort (13) intérieure montée de façon coaxiale sur le boîtier d'instrument (2).

2. Instrument optique selon la revendication 1, **caractérisé en ce que** chacun des éléments de ressort (11) se trouve directement contre l'élément d'étanchéité (9) associé.

3. Instrument optique selon l'une des revendications 1 à 2, **caractérisé en ce que** les éléments d'étanchéité (9) sont formés en tant que joints toriques (9).

4. Instrument optique selon la revendication 1, **caractérisé en ce que** la fenêtre d'extrémité (5) se situe à l'extrémité proximale du boîtier d'instrument (2) qui présente une coque oculaire (10) dans laquelle est formée une rainure (14) radialement périphérique dans laquelle les rondelles de ressort (12) sont montées sur un côté.

5. Instrument optique selon l'une des revendications 1 à 4, **caractérisé en ce que** la fenêtre d'extrémité (5) est formée en tant que verre de recouvrement (7) et/ou lentille d'extrémité (6).
